# FASCICULE DE BREVET EUROPEEN

(11) **EP 4 486 278 B1**
(45) Date de publication et mention de la délivrance du brevet: **09.12.2026**
(21) Numéro de dépôt: 23711524.1
(22) Date de dépôt: 01.03.2023
(51) Int. Cl.: A61F 13/15, A61F 13/02

(54) **PANSEMENT MODULABLE**
EINSTELLBARER VERBAND
ADJUSTABLE DRESSING

(30) Priorité: 03.03.2022 FR 2201852
(43) Date de publication de la demande: 08.01.2025
(73) Titulaire: URGO RECHERCHE INNOVATION ET DEVELOPPEMENT, 21300 Chenôve (FR)
(72) Inventeur: BAHUON, Manuelle, 21000 DIJON (FR); LEFEUVRE, Solène, 21220 EPERNAY-SOUS-GEVREY (FR); PASQUINET, Laurent, 21800 CRIMOLOIS (FR); FOUROT-BENOSMAN, Amélie, 21560 ARC SUR TILLE (FR)
(74) Mandataire: Cabinet Beau de Loménie
(86) Numéro de dépôt international: PCT/FR2023/050275
(87) Numéro de publication internationale: WO 2023/166262

(56) Documents cités:
- EP-B1- 3 515 386
- WO-A1-2012/140377
- WO-A1-2017/039668
- US-A1- 2017 224 866

## Description

### Domaine Technique

La présente invention est relative à un pansement modulable pour favoriser la cicatrisation d'une escarre notamment au niveau du sacrum.

### Technique antérieure

Les escarres, appelées aussi ulcères de pression, sont des plaies qui résultent d'une hypoxie tissulaire, c'est-à-dire une absence d'apport d'oxygène des tissus superficiels et ou profonds en général au-dessus d'une proéminence osseuse. Les escarres les plus courants sont ainsi présentes au niveau du sacrum, du talon et des coudes. Ils apparaissent souvent sur des patients hospitalisés qui bougent peu et sont dénutris. Les tissus au-dessus de la proéminence osseuse subissent une combinaison de forces de pression, de cisaillement et de friction qui altèrent les tissus mous et conduisent à une nécrose de ces tissus mous créant ainsi l'ulcère de pression. Si les contraintes appliquées sont importantes une telle nécrose peut apparaitre en quelques minutes seulement. Les escarres ont été classées en 1989 par la « National Pressure Ulcer Advisory Panel » en 4 stades en fonction de leur gravité.

Pour éviter l'apparition de ces ulcères au niveau du sacrum on a recours à des matelas spécifiques pour soulager la pression appliquée et à des modifications régulières de la position des patients dans leur lit.

De nombreux pansements sont aussi couramment utilisés dans le cadre de la prévention des escarres du sacrum et du talon. Leur mécanisme d'action est généralement attribué à leurs propriétés d'absorption des chocs et de répartition des pressions appliquées. Ces pansements intègrent dans leurs structures des couches de matériaux spécifiquement choisis pour obtenir ces propriétés. A titre d'exemples de tels pansements, on peut citer ceux décrits dans la demande de brevet WO2017 / 220405 lesquels comprennent une couche anisotropique spécifique pour prévenir une escarre du sacrum.

Toutefois, dès lors qu'un ulcère s'est déjà formé, les techniques et pansements précités s'avèrent souvent insuffisants et des recherches ont été réalisées pour disposer d'un pansement qui permettrait de favoriser la cicatrisation des plaies d'escarres superficielles du type 1, 2 voire 3 afin d'éviter qu'elles s'aggravent et atteignent le stade beaucoup plus grave de niveau 4.

De plus, il serait particulièrement intéressant, notamment pour faciliter l'utilisation par le personnel soignant, de disposer d'un pansement dérivé d'un pansement classique, sans modification substantielle de sa structure et qui pourrait être facilement adapté selon le patient, la taille de sa plaie et sa position.

Dans ce contexte, il a été proposé, dans la demande de brevet EP 164 319, un pansement comprenant une couche modulable conçue pour soulager la pression appliquée au niveau de la plaie, généralement désignée par l'expression « couche de décharge de la plaie ». Un tel pansement est commercialisé depuis de nombreuses années par la société Coloplast sous la dénomination Comfeel Plus Pressure Relief.

Ce pansement est constitué, en partant de sa face destinée à venir au contact de la plaie en position d'utilisation, d'une couche absorbante unique recouverte d'un film de protection sur lequel est fixée une couche de décharge de la plaie. Cette couche est prédécoupée pour définir un ensemble de parties contigües indépendamment détachables, les parties détachables se trouvant en vis-à-vis de la zone de la plaie où s'applique habituellement la plus forte pression, étant retirées, en position d'utilisation, afin de supprimer ou diminuer cette pression.

Grâce au caractère modulable de sa couche de décharge de la plaie, ce pansement est adaptable à toute taille et zone du corps.

Le matériau constituant la couche de décharge de la plaie dans le pansement Comfeel Plus Pressure Relief est une mousse hydrophobe qui présente l'avantage ne pas absorber de liquide ou d'en absorber seulement de faibles quantités et d'éviter ainsi un risque de modifications de ses propriétés par une absorption importante de liquides. De plus, la propagation des liquides à l'intérieur de ces mousses hydrophobes est très limitée, contrairement à ce qui se produit avec des mousses hydrophiles. Ceci est particulièrement intéressant dans le cas d'une escarre du sacrum, la mousse empêchant ainsi toute contamination de la plaie en cas de fuites urinaires susceptibles d'intervenir avec des patients alités.

Dans la demande de brevet EP164 319 il est en outre précisé (page 6 lignes 24 à 30) que les mousses peuvent être comprimées. Cette compression et la nature hydrophobe de la mousse permettent de diminuer le nombre de cellules ouvertes et leur taille réduisant ainsi leur nombre en surface et au sein de la mousse et le risque corrélatif de contact avec un liquide extérieur.

On peut aussi noter que dans la demande EP164 319 les mousses décrites en tant que matériau constitutif de la couche de décharge de la plaie sont caractérisées par leur épaisseur, leur nature hydrophobe, leur densité et leur module d'élasticité (page 4 lignes 11 à 25).

Le pansement décrit dans la demande EP164 319 et illustré par le produit Comfeel Plus Pressure Relief a apporté une première réponse au besoin de disposer d'un pansement permettant de réduire les problèmes résultant de la pression appliquée sur la plaie chez des patients immobilisés dans leur lit. Le document EP3515386 décrit un autre pansement modulable pour le traitement d'escarres.

Cependant, il apparait que cette réponse reste encore insuffisante dans le traitement des escarres, dans la mesure où elle ne prend en compte qu'une partie des phénomènes responsables de la création et l'évolution des escarres, à savoir les phénomènes résultant de la pression exercée sur la plaie.

En particulier, il a été observé que le pansement Comfeel Plus Pressure Relief ne gère pas de façon optimale les conséquences du cisaillement qui est un paramètre également important dans la création des escarres. Ainsi, si ce pansement permet de réduire efficacement la pression exercée au niveau de la plaie, on a observé que son utilisation s'accompagne d'un report de pression sur les parties périphériques de la plaie rendant plus difficile la cicatrisation.

En outre, le pansement décrit dans la demande EP164 319 et illustré par le produit Comfeel Plus Pressure Relief ne comprend qu'une seule couche absorbante très particulière incorporant des particules d'hydrocolloïdes dispersées dans un adhésif, qui s'avère en pratique limitée en termes de capacité d'absorption et de durée d'utilisation.

Enfin, les mousses hydrophobes dont l'utilisation est préconisée dans la demande EP164 319 sont des produits dont la fabrication est de plus en plus coûteuse pour répondre aux normes imposées aux produits qualifiés de « médicaux » notamment en raison des conditions exigées en termes d'hygiène des installations et de contrôle de fabrication.

Il existe donc un besoin non satisfait à ce jour de disposer d'un pansement d'une nouvelle conception, qui prenne en compte tous les phénomènes impliqués dans la formation des escarres et de leur évolution vers un stade de gravité plus avancé (stade 3 ou 4), disposant d'une capacité d'absorption plus élevée et d'une durée d'utilisation plus longue, et qui puisse être fabriqué à l'échelle industriel sans surcoût important.

A la suite de recherches intensives dans la compréhension et la modélisation des phénomènes impliqués dans la création des escarres, il a été découvert, et ceci constitue le fondement de la présente invention, qu'il était possible de répondre à ce besoin et de proposer un pansement modulable optimisé avec une couche de décharge de la plaie qui prenne en compte toutes les contraintes applicables et une structure générale en adéquation avec le marché actuel des pansements.

### Exposé de l'invention

Ainsi, selon un premier aspect, la présente invention est donc relative à un pansement modulable défini dans la revendication 1 annexe utilisable dans le traitement d'une plaie et destiné notamment à traiter une escarre, en particulier une escarre du sacrum

Dans le cadre de la présente description, la mousse de décharge recouverte d'un film de protection est également désignée par l'expression « élément de décharge ».

Dans le cadre de la présente invention on utilise des mousses hydrophiles qui présentent une densité comprise entre 70 et 250 kg/m³ et de préférence entre 90 et 220 kg/m³.

L'épaisseur de la couche de décharge est de 5 mm au minimum pour obtenir l'effet recherché mais pourra être supérieure, jusqu'à 10 mm. Toutefois on préférera utiliser des mousses dont l'épaisseur est inférieure à 8 mm pour éviter un pansement trop épais qui pourrait gêner le patient. Avantageusement, l'épaisseur de la couche de décharge sera comprise entre 5 et 7 mm d'épaisseur.

Parmi ces mousses on préférera celles qui présentent une complaisance recouvrable supérieure ou égale à 1. 10⁻⁵ Pa⁻¹, de préférence comprise entre 1.10⁻⁵ Pa⁻¹ et 4.10⁵ Pa⁻¹.

Selon une caractéristique particulière de l'invention, le film de protection perméable à la vapeur d'eau et imperméable aux liquides précités présente un coefficient de friction compris entre 0,8 et 1.2.

Selon une autre caractéristique particulière, le pansement de l'invention présente un coefficient de friction, mesuré côté film, inférieur à 1 et de préférence compris entre 0,4 et 0,8.

Selon encore une autre caractéristique particulière de l'invention le pansement comprend au moins deux couches absorbantes choisies parmi les mousses hydrophiles absorbantes, les non tissés absorbants contenant des particules super absorbants, une couche comprenant des particules de polymères super absorbants entre deux non tissés et les non tissés à base de fibres super absorbantes.

Selon un deuxième aspect, la présente invention concerne un élément de pansement défini dans la revendication 10 annexe notamment utilisable dans le traitement d'une escarre, en particulier d'une escarre du sacrum, comprenant une mousse de décharge comportant un ensemble de parties contigües indépendamment détachables, lesdites parties détachables destinées à se trouver en vis-à-vis de la plaie ou d'une partie de celle-ci, en position d'utilisation, étant retirés afin de réduire ou supprimer la pression susceptible de s'exercer sur cette zone[0029] Selon un mode de réalisation préféré, le pansement modulable de l'invention est caractérisé en ce que :
- l'une au moins des couches absorbantes est choisie parmi une couche de mousse absorbante de polyuréthane hydrophile, de préférence de 1,5 à 5 mm d'épaisseur ; une couche de non tissé de fibres absorbantes, en particulier de cellulose, de rayonne, de viscose, incorporant des particules de polymère super absorbant, en particulier des polymères acryliques ou leurs sels ; une couche de non tissé de fibres super absorbantes ; une couche comprenant des particules de polymères super absorbants entre deux non tissés ;
- ladite mousse de décharge est une mousse hydrophile de polyuréthane présentant une épaisseur comprise entre 5 et 10 mm et une densité comprise entre 70 et 250 kg/m³ ; et
- ladite couche de mousse de décharge est recouverte d'un film de protection perméable à la vapeur d'eau et imperméable aux liquides présentant une épaisseur de 10 à 40 µm, et de préférence un coefficient de friction compris entre 0,8 et 1,2.

Selon une caractéristique particulière, le film précité est un film en polyuréthane.

Selon une autre caractéristique particulière, l'une des couches absorbantes précitées est un non tissé de fibres absorbantes incorporant des particules de polymère super absorbant dans une proportion comprise entre 1 et 70 % en poids, de préférence de 25 à 55 % en poids, du poids total du non tissé.

Selon une autre caractéristique particulière, l'une au moins des couches absorbantes du pansement est un non tissé ou une couche comprenant des particules de polymères super absorbants entre deux non tissés présentant une épaisseur comprise entre 0,5 et 3 mm et/ou un grammage compris entre 200 et 800 g/m² et/ou une absorption supérieure à 5 000 g/m², de préférence supérieure ou égale à 15 000 g/m².

Selon un mode de réalisation préféré de l'invention, le pansement comprend :
- une couche de mousse absorbante hydrophile telle que définie précédemment sur laquelle est disposé un non tissé de fibres super absorbantes ; ou
- une couche de mousse absorbante hydrophile telle que définie précédemment sur laquelle est(sont) disposé(s) un(ou deux) non tissé(s) de fibres absorbantes incorporant des particules de polymère super absorbant ou une couche comprenant des particules de polymères super absorbants entre deux non tissés ; ou
- un non tissé à base de fibres super absorbantes sur lequel est(sont) disposé(s) un(ou deux) non tissé(s) de fibres absorbantes incorporant des particules de polymère super absorbant ou une couche comprenant des particules de polymères super absorbants entre deux non tissés.

L'invention sera mieux comprise et d'autres caractéristiques apparaîtront plus clairement à la lecture de la description explicative qui va suivre faite en référence aux dessins annexés dans lesquels :

### Brève description des dessins

[Fig. 1] La figure 1 est un schéma illustrant le principe des mesures qui ont été réalisées avec un rhéomètre ;
[Fig. 2] La figure 2 représente une mousse de décharge avec ses parties détachables selon un mode de réalisation de l'invention ;
[Fig. 3] La figure 3 illustre le fichier Excel qui a été utilisé dans le cadre des tests réalisés pour aider au positionnement des pansements à tester sur la zone du sacrum où s'appliquent les plus fortes pressions ;
[Fig. 4] La figure 4 est une image représentant la répartition des pressions s'exerçant sur le sacrum d'un volontaire sain sans pansement et avec le pansement Comfeel Plus Pressure Relief°;
[Fig. 5] La figure 5 est une image semblable à la figure 4 représentant la répartition des pressions s'exerçant sur le sacrum d'un volontaire sain sans pansement et avec un pansement modulable selon l'exemple M8 de l'invention ;
[Fig. 6] La figure 6 est une image semblable à la figure 4 représentant la répartition des pressions s'exerçant sur le sacrum d'un volontaire sain sans pansement et avec un pansement modulable selon l'exemple M9 de l'invention ;
[Fig. 7] La figure 7 est une image semblable à la figure 4 représentant la répartition des pressions s'exerçant sur le sacrum d'un volontaire sain sans pansement et avec un pansement modulable selon l'exemple M10 de l'invention ;
[Fig 8] La figure 8 illustre les déformations maximales de cisaillement (selon la définition de Green-Lagrange) dans les tissus mous de la zone centrale et de la zone perilésionnelle d'une plaie de stade 2 dans trois situations :
   - sans pansement,
   - avec le pansement de l'exemple P4 de l'invention et
   - avec le pansement modulable de l'exemple M8 de l'invention ;
[Fig. 9] La figure 9 illustre les déformations maximales de cisaillement (selon la définition de Green-Lagrange) dans les tissus mous sur toute la zone visée (zone centrale et zone périlésionnelle de la plaie) sans pansement et avec le pansement modulable de l'exemple M8 de l'invention ;
[Fig. 10] La figure 10 représente schématiquement le siège utilisé dans les essais visant à visualiser les pressions qui s'appliquent sur le sacrum d'un sujet sans plaie ;
[Fig. 11] La figure 11 représente schématiquement un coussin d'aide à la prévention d'escarres utilisé avec le siège représenté à la figure 10 ;
[Fig. 12] La figure 12 représente schématiquement un testeur équipé d'une nappe textile de pression pour les essais visant à visualiser les pressions qui s'appliquent sur le sacrum d'un sujet sans plaie.

### Description des modes de réalisation

A l'instar du pansement généralement décrit dans la demande EP 164 319, la mousse de décharge du pansement selon l'invention comporte un ensemble de parties contigües indépendamment détachables qui peuvent être formées par tout moyen permettant de réaliser des amorces de détachement, comme en particulier par découpe laser.

Ces parties détachables peuvent être de configurations variées. Il peut ainsi s'agir de triangles, carrés, rectangles, hexagones juxtaposés en forme de mosaïques ou encore de disques ou ovales concentriques.

La surface de chacune de ces parties détachables est généralement comprise entre 0.5 et 2.5 cm⁻², de préférence comprise entre 1 et 2 cm⁻².

Lors de l'utilisation, les parties détachables destinées à se trouver en vis-à-vis des zones de la plaie où s'exercent habituellement les plus fortes pressions sont retirées par le personnel soignant, afin de réduire ou supprimer la pression susceptible de s'exercer dans ces zones.

Le nombre de parties ainsi détachées dépend bien entendu de l'étendue de la plaie ou de la zone susceptible de conduire à une plaie, ainsi que de la surface de chaque partie détachée et sera généralement compris entre 15 et 45, de préférence entre 20 et 35 pour une surface comprise environ entre 10 et 30 cm².

A la différence du pansement généralement décrit dans la demande EP 164 319 et illustré par le produit Comfeel Plus Pressure Relief, la mousse formant la couche de décharge du pansement selon l'invention est hydrophile et recouverte par un film continu imperméable aux liquides mais perméable à la vapeur d'eau.

Les inventeurs ont en effet vérifié que l'utilisation, en tant que couche de décharge, d'une mousse hydrophile pouvait conduire à une absorption de liquides susceptibles d'interagir de façon néfaste avec la plaie à traiter et ont constaté que l'utilisation d'un film de protection continu, imperméable aux liquides mais perméable à la vapeur d'eau permettait de façon particulièrement avantageuse de limiter la capacité d'absorption de la mousse, sans altérer la capacité d'évaporation des liquides provenant de la plaie.

Ce film peut être choisi parmi ceux utilisés de façon courante pour la fabrication des pansements absorbants.

Selon un mode de réalisation préféré, on choisira un film, de préférence de polyuréthane, présentant un taux de transmission de vapeur d'eau (ou MVTR pour Moisture Vapor Transmission Rate) supérieur ou égal à 7 000 g/m /24 heures, de préférence encore supérieur ou égal à 10 000 g/m /24 heures. De façon encore préférée, on choisira un film en polyuréthane tel que par exemple, le film commercialisé par la société Covestro sous la référence 9101 T7.

Selon une caractéristique particulière, le film présente une épaisseur de l'ordre de 10 à 40 micromètres. Il a été découvert qu'à cette épaisseur, le film ne modifie pas les caractéristiques avantageuses de la mousse en termes de réduction des forces de pression et de cisaillement appliquées à la plaie.

Il a en outre été constaté qu'un tel film présente une faible capacité de friction ce qui permet, de façon avantageuse, de diminuer les risques de friction de la mousse ou de formation de plis du drap sur lequel repose le patient.

Ce film est fixé sur la face de la mousse de décharge destinée à être la plus éloignée de la plaie en position d'utilisation, de préférence avant la formation des parties détachables précitées.

La fixation du film sur la mousse de décharge peut être réalisée par la mise en œuvre de techniques classiques d'assemblage, comme par exemple par l'enduction d'un adhésif sur la mousse ou le film, de préférence par une enduction partielle pour faciliter la respirabilité ; à l'aide d'un adhésif double face ; ou encore par liaison thermique ou par flambage.

Selon un mode de réalisation particulièrement avantageux, le film de protection présente un coefficient de friction compris entre 0,8 et 1.2 et le pansement selon l'invention pourvu de ce film, présente un coefficient de friction, mesuré côté film, inférieur à 1 et de préférence compris entre 0,4 et 0,8.

La mesure du coefficient de friction est classique et son principe est décrit dans la norme EN ISO 8295. Elle consiste à mesurer, à l'aide d'un dynamomètre électronique, la force nécessaire pour déplacer sur un grand plateau, un patin carré en acier de 63 x 63 mm de 200 grammes recouvert du pansement avec sa couche de décharge et tiré à la vitesse de 100 mm/minute. Le coefficient de friction est le rapport entre la force de traction mesurée par le dynamomètre et exprimée en Newtons et le poids du patin exprimé en Newtons (soit 1,98 N tel que défini dans la norme).

Toutes les mousses hydrophiles ne peuvent être employées dans le cadre de l'invention et les inventeurs ont mené des recherches intensives afin de définir les caractéristiques que ces mousses doivent posséder pour permettre une décharge de la plaie efficace avec un pansement qui comprend au moins deux couches absorbantes.

A cet effet, les inventeurs ont notamment étudié non seulement le comportement de l'élément de décharge seul (mousse et film) mais également celui du pansement incorporant l'élément de décharge dans les conditions les plus proches de la réalité, c'est-à-dire en les soumettant aux différentes contraintes possibles.

Plus précisément, les inventeurs ont utilisé une approche totalement nouvelle prenant en compte toutes les contraintes que le pansement subit de façon simultanée, lors de l'apparition ou de la présence d'une escarre, à savoir une pression importante, un cisaillement mais également les mouvements du patient car même alité le patient n'est pas immobile. Ces mouvements (ou micromouvements) se produisent par exemple lors du changement de position du patient ou de glissement dans le lit.

Cette notion de mouvement, qui n'avait jamais été prise en compte jusqu'à présent dans l'état de la technique, est importante et la mise au point d'un pansement efficace dans le traitement d'une escarre ne peut être fondée sur la seule base des résultats de mesures réalisées dans un scénario d'immobilité totale. En effet, comme indiqué précédemment, des sollicitations même très courtes conduisant à un choc, un frottement et/ou un cisaillement peuvent impacter les tissus mous et conduire à l'apparition d'une escarre ou dégrader une escarre déjà existante.

Les inventeurs ont donc recherché une technique permettant d'appliquer simultanément des contraintes de pression et de cisaillement dans un mode dynamique afin de définir quelles sont les propriétés qu'un pansement et/ ou un élément de décharge doivent présenter pour permettre de traiter de la meilleure façon une escarre, et en particulier une escarre du sacrum.

Les inventeurs ont ainsi découvert que des mesures de rhéologie oscillatoire à l'aide d'un rhéomètre (qui seront décrites en détail par la suite) permettent de répondre à cet objectif et de mettre au point de nouveaux pansements aptes à favoriser la cicatrisation d'une escarre.

Les résultats de ces études ont ainsi montré qu'un pansement particulièrement adapté aux traitement des escarres doit comprendre :
- au moins deux couches absorbantes et
- une couche de mousse de décharge hydrophile, recouverte sur sa face la plus éloignée de la plaie, en position d'utilisation, d'un film de protection perméable à la vapeur d'eau et imperméable aux liquide ; ladite couche de mousse et son film comportant un ensemble de parties contigües détachables ;
et que ladite couche mousse doit en outre présenter une épaisseur d'au moins 5 mm et un module de cisaillement complexe G* compris :
- entre 20 et 70 kPa et de préférence entre 30 et 60 kPa à une fréquence de 0,1 Hz
- entre 30 et 90 kPa et de préférence entre 35 et 80 kPa à une fréquence à une fréquence de 1 Hz
- entre 40 et 100 kPa et de préférence entre 40 et 90 kPa à une fréquence de 10 Hz.

Avantageusement, la couche de mousse de décharge précitée présente en outre une propriété dite de « complaisance recouvrable » supérieure ou égale à 1. 10⁻⁵ Pa⁻¹ dans des conditions de mesure définies.

Ces différents aspects de l'invention vont être détaillés dans ce qui suit.

Pour traiter efficacement une escarre, un pansement doit être capable de subir, « absorber » et « dissiper » l'énergie qu'il reçoit de contraintes simultanées de pression et de cisaillement.

Afin d'évaluer la capacité d'un élément de décharge (mousse et film) ou d'un pansement incorporant un tel élément de répondre à cette exigence, les inventeurs ont utilisé un rhéomètre, appareil classique de laboratoire, et mis au point une technique de mesure spécifique.

Plus précisément cette technique consiste à appliquer simultanément sur le pansement ou l'élément de décharge, une pression que l'on calibre pour correspondre à la pression correspondant à une escarre ainsi qu'une contrainte de cisaillement et ceci à une température de l'ordre de celle d'un patient alité. Ce test est réalisé à différentes fréquences qui vont illustrer les mouvements du patient et présente de ce fait un caractère oscillatoire.

Il a été constaté que la fréquence de balayage la plus adaptée se situe dans la plage allant de 0,01 Hz à 10 Hz. Cette plage de fréquences est en effet la plus représentative de l'ensemble des fréquences caractéristiques des mouvements d'un patient alité. Ainsi, dans le cas du traitement d'une escarre du sacrum, le pansement fixé entre le matelas et les tissus biologiques du patient est soumis à un ensemble de mouvements allant de fréquences très faibles, de l'ordre de 0,01 Hz voire 0,1 Hz (représentatives d'une quasi- immobilité) à des fréquences comprises de 1 Hz à 10 Hz (représentatives de mouvements plus significatifs tels que les mouvements du corps, les micro- glissements, les changements de position, la respiration, etc.).

Ce test a ainsi permis, à partir du couple pression/cisaillement appliqué de façon oscillatoire, de caractériser le comportement viscoplastique du pansement ou de l'élément de décharge, lequel se traduit par le calcul de 3 paramètres :
- le module élastique de cisaillement G', souvent qualifié de module de conservation, qui caractérise la rigidité et le comportement élastique en cisaillement du matériau testé ;
- le module de perte ou de dissipation en cisaillement G " qui caractérise l'énergie dissipé par le matériau en cisaillement ;
- le module de cisaillement dynamique, également dénommé ici module de cisaillement complexe G*, qui détermine la dissipation mécanique du pansement ou de la couche de décharge, défini par l'équation : [Math. 1] $G * = \sqrt{{G}^{′ 2} + {G}^{" 2}} .$

Il a été constaté que le module de cisaillement dynamique G* évaluée aux trois fréquences choisies de 0,1 ; 1 et 10 Hz est le mieux adapté pour caractériser le comportement en dynamique de l'élément de décharge ou du pansement testé et déterminer quels sont les pansements et les éléments de décharge les plus efficaces dans le but poursuivi.

Les premiers tests réalisés par les inventeurs ont montré que cette mesure, appliquée à un pansement pourvu ou non d'un élément de décharge (mousse + film de protection), est discriminante et permet d'observer des différences entre les produits.

Ces tests ont notamment permis de quantifier le comportement des pansements testés et de classer les pansements comprenant au moins 2 couches absorbantes en deux grandes catégories :
- ceux dont au moins une couche absorbante est une mousse absorbante hydrophile ; et
- ceux qui ne possèdent aucune mousse absorbante hydrophile.

Cette même technique a également permis d'étudier le comportement, au cours du temps, d'une mousse de décharge qui a subi l'action simultanée d'une pression et d'un cisaillement. Dans ce cas le protocole a été légèrement modifié et consiste :
- d'une part à appliquer une contrainte de pression comme précédemment ; et
- d'autre part à appliquer une contrainte de cisaillement de 100 Pa pendant 100 minutes puis à supprimer cette contrainte pendant 30 minutes afin de permettre à l'échantillon de prendre un nouvel équilibre mécanique.

Ce test a permis de mesurer plusieurs propriétés qui caractérisent le comportement de la mousse de décharge.

En premier lieu, il permet de mesurer la déformation instantanée de la mousse (ou de l'élément de décharge) consécutive à l'application de la pression et de la contrainte de cisaillement ainsi que sa déformation rémanente, c'est-à-dire sa capacité à reprendre une partie de sa forme initiale quand la contrainte de cisaillement a cessé.

Il est en effet important de s'assurer que l'élément de décharge choisi reprend bien une partie de ses propriétés initiales pour résister à la succession de contraintes qu'il va subir en condition réelle d'utilisation. Ainsi, à partir des mesures de déformations ainsi réalisées les inventeurs ont utilisé la possibilité du rhéomètre, de calculer directement un paramètre dénommé ici « complaisance recouvrable » qui permet de caractériser et de quantifier l'aptitude de l'élément de décharge testé à reprendre au moins une partie de ses propriétés initiales. Cette complaisance recouvrable directement calculée par le rhéomètre est la différence entre les valeurs mesurées par le rhéomètre (et exprimées sans unités) de la déformation instantanée et de la déformation rémanente divisée par la contrainte appliquée ici 100Pa.

Tous ces tests ont permis d'étudier différents pansements et éléments de décharge, de les classifier et de déterminer, en fonction des valeurs obtenues pour ces caractéristiques, ceux qui permettent de parvenir à l'objectif recherché.

Dans ce contexte, les inventeurs ont également mis au point une technique d'évaluation de l'efficacité des produits classifiés permettant de vérifier les résultats obtenus. Une étude réalisée sur des volontaires sans plaie au niveau du sacrum a permis de sélectionner les mousses de décharge remplissant les propriétés recherchées pour les deux catégories de pansements déterminées lors du premier test.

L'ensemble de ces études a permis de montrer que les mousses de décharge hydrophiles permettant de traiter au mieux les escarres, en particulier les escarres du sacrum, doivent posséder deux caractéristiques essentielles qui sont une épaisseur d'au moins 5 mm et un module de cisaillement complexe G* compris :
- entre 20 et 70 kPa et de préférence entre 30 et 60 kPa à une fréquence de 0,1 Hz
- entre 30 et 90 kPa et de préférence entre 35 et 80 kPa à une fréquence à une fréquence de 1 Hz
- entre 40 et 100 kPa et de préférence entre 40 et 90 kPa à une fréquence de 10 Hz.

De préférence ces mousses hydrophiles présentent une densité comprise entre 70 et 250 Kg/m³. Dans le cadre de l'invention, il s'agit de mousses de polyuréthane.

Enfin des études complémentaires sur le cisaillement ont été réalisées à l'aide de la modélisation. A la connaissance du déposant l'action du cisaillement sur les tissus mous n'a jamais été évaluée quantitativement.

La modélisation par éléments finis est une technique couramment utilisée pour étudier le biomécanisme de création des escarres mais les résultats habituellement obtenus permettent uniquement d'établir un comparatif entre plusieurs pansements.

Afin de vérifier l'efficacité d'un pansement modulable selon l'invention sur le sacrum, une nouvelle technique de modélisation a donc été développée pour quantifier, à l'aide des équations de Green Lagrange, l'action du cisaillement sur les tissus mous sur un sujet sans plaie. Partant des résultats obtenus, on a alors incorporé dans ce modèle une plaie de type 2 et ainsi chiffré de la même façon l'action du cisaillement sur la plaie et son pourtour. Cette simulation avec une plaie a permis de confirmer l'action positive des mousses de décharge sélectionnées en montrant notamment que ces mousses permettent de réduire de façon significative les effets du cisaillement non seulement dans la zone de la plaie mais également à sa périphérie.

L'ensemble de ces études et les résultats obtenus sont rapportés dans la partie expérimentale de la présente demande.

Les pansements complexes qui sont utilisés dans le cadre de la présente invention comprennent au moins deux couches absorbantes superposées, l'épaisseur de chaque couche étant avantageusement choisie en fonction de la capacité d'absorption souhaitée.

Ces couches absorbantes, couramment employées dans les pansements modernes, peuvent être choisies parmi les hydrogels, les mousses absorbantes hydrophiles ou hydrophobes rendues hydrophiles et les matériaux textiles absorbants.

Une mousse absorbante peut- être avantageusement choisie parmi les mousses absorbantes hydrophiles et les mousses hydrophobes rendues hydrophiles par l'incorporation de particules absorbantes.

Des mousses absorbantes hydrophiles et leur procédé de fabrication, bien connus de l'homme de l'art, sont par exemple décrits dans les demandes de brevet WO 96/16099, WO 94/29361, WO 93/04101, EP 420 515, EP 392 788, EP 299 122, et WO 2004/074343. De telles mousses correspondent par exemple à la référence MCF03 commercialisée par la société AMS, ou à la référence 562-B commercialisée par la société RYNEL.

Selon un mode de réalisation préféré de l'invention, l'une des couches absorbantes du pansement est une mousse de polyuréthane hydrophile, de préférence une mousse de polyuréthane de 1,5 à 5 mm d'épaisseur.

Les matériaux textiles absorbants susceptibles d'être utilisés dans le cadre de l'invention pour constituer la partie absorbante du pansement peuvent être des tricots, des tissés ou des non-tissés absorbants et en particulier des non-tissés absorbants couramment employés dans le domaine des pansements ou de l'hygiène.

Un non tissé absorbant peut être choisi parmi les non tissés de fibres absorbantes (telles que des fibres de cellulose, de rayonne ou de viscose) comprenant des particules de polymère super absorbant, et les non-tissés faits au moins en partie de fibres super absorbantes.

Le non tissé peut être avantageusement obtenu par la méthode de fabrication par voie sèche connue sous le nom de voie aérodynamique ou "airlaid". Tous les modes de liages couramment utilisés dans la technologie des non tissés pourront être employés pour fabriquer le non tissé absorbant : liage par enduction spray de latex, liage par incorporation de fibres ou de poudres thermoliantes puis traitement thermique, liage par association de ces deux techniques, liage par simple compression des fibres. Ce dernier mode de liage, ne faisant pas appel à l'incorporation de matériaux thermoliants ou de latex, sera préféré.

Selon une variante l'une des couches absorbantes du pansement peut être une couche comprenant des particules de polymères super absorbants entre deux non tissés.

Selon un mode de réalisation de l'invention, l'une des couches absorbantes du pansement est un non tissé de fibres absorbantes incorporant des particules de polymère super absorbant dans une proportion comprise entre 1 et 70% en poids, de préférence de 25 à 55% en poids, du poids total du non tissé. Le polymère super absorbant peut être choisi parmi les polymères acryliques ou leurs sels tels que les polyacrylates de sodium.

Selon un autre mode de réalisation de l'invention, l'une des couches absorbantes du pansement est un non tissé à base de particules de polymère super absorbant et de fibres de cellulose préparé sans l'incorporation de matériaux thermoliants ou de latex, qui sera recouvert sur chacune de ces faces par un voile cellulosique.

Selon une variante de réalisation, le non tissé absorbant est constitué de deux voiles cellulosiques entre lesquels sont incorporés des particules de polymère super absorbant seules ou en association avec des liants.

Selon un autre mode de réalisation de l'invention, l'une des couches absorbantes du pansement est un non tissé absorbant fait au moins en partie de fibres super absorbantes.

Dans le cadre de la présente invention, on préfère utiliser, pour l'une au moins des couches absorbantes du pansement, un non tissé ou une couche comprenant des particules de polymères super absorbants entre deux non tissés présentant une épaisseur comprise entre 0,5 et 3 mm, et/ou un grammage compris entre 200 et 800 g/m² et/ou une absorption supérieure à 5000 g/ m², de préférence encore supérieure ou égale à 15000 g/ m² ; l'absorption étant mesurée selon la norme EDANA 440.1.99.

Des non tissés absorbants appropriés sont par exemple commercialisés par la société EAM Corporation sous la référence Novathin ^{®}.

Selon un mode de réalisation préféré de l'invention le pansement comprend :
- une couche de mousse absorbante hydrophile telle que définie précédemment sur laquelle est disposée un non tissé de fibres super absorbantes ; ou
- une couche de mousse absorbante hydrophile telle que définie précédemment sur laquelle est(sont) disposé(s) un (ou deux) non tissé(s)de fibres absorbantes incorporant des particules de polymère super absorbant ou une couche comprenant des particules de polymères super absorbants entre deux non tissés ; ou
- un non tissé à base de fibres super absorbantes sur lequel est disposé un (ou deux) non tissé(s) de fibres absorbantes incorporant des particules de polymère super absorbant une couche comprenant des particules de polymères super absorbants entre deux non tissés.

Selon une caractéristique particulière de la présente invention, le pansement comprend en outre, dans au moins l'une de ses couches absorbantes, de préférence celle venant au contact de la plaie, un composé pour favoriser ou optimiser la cicatrisation de la plaie. A titre de tel composé préféré, on peut citer un sel d'argent ou bien encore le sel de potassium de sucrose octa sulfate.

De telles structures de la partie absorbante du pansement selon l'invention correspondent notamment aux pansements commercialisés par la société Urgo Medical sous les dénominations URGOTUL^{®} Absorb Border et URGOSTART PLUS Border^{®} qui sont respectivement décrits dans les demandes de brevet WO2012/140377 et WO 2012/140378 auxquelles l'homme de métier pourra se reporter.

L'invention sera illustrée par les exemples non limitatifs suivants.

Mesure du module de cisaillement complexe (ou module de cisaillement dynamique) des pansements ou de l'élément de décharge La mesure du module de cisaillement complexe_est effectuée selon la méthode suivante.

Le pansement modulable ou la mousse de décharge seule ou complexée au film imperméable est soumis à un couple pression/cisaillement à l'aide d'un rhéomètre DHR2 commercialisé par la société TA Instrument équipé d'un plan Peltier pour la régulation de température.

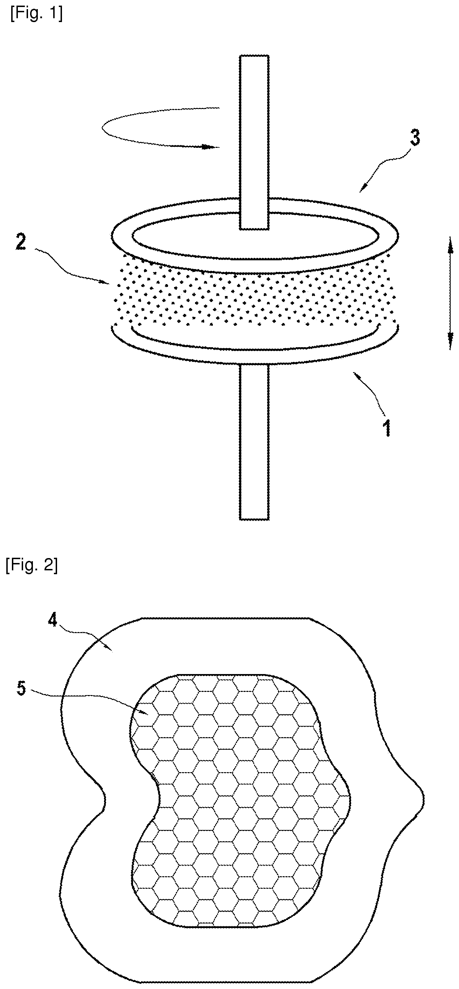

Le principe de la mesure est illustré sur la figure 1 où le chiffre de référence 2 représente l'échantillon testé et les chiffres de référence 1 et 3 représentent respectivement les plateaux inférieur et supérieur du rhéomètre.

Dans ce test, on a appliqué simultanément à l'échantillon testé une contrainte de compression axiale d'un niveau comparable à celle rencontrée dans le domaine des appuis sacrums dans les pathologies d'escarres soit d'environ 28 kPa ainsi qu'une déformation en torsion mimant le cisaillement. Le niveau de compression axiale de 28 kPa est transformé en une force axiale rapportée à la surface du plateau mobile 3 de 1,4 N (flèche verticale) et le cisaillement est appliqué par une déformation de 1% via une rotation du plateau mobile (flèche circulaire).

On a appliqué ce couple pression/cisaillement durant des durées variables qui correspondent aux fréquences choisies soit 10 secondes pour 0,1Hz, 1 seconde pour 1Hz et 0,1 seconde pour 10Hz.

Pour être représentatif de la taille de la partie centrale du pansement qui comprend les couches absorbantes superposées, on a choisi un plateau mobile de 25 mm de diamètre.

A l'aide d'un emporte-pièce circulaire de ce diamètre on a découpé en leur centre plusieurs échantillons du pansement ou de la mousse à analyser.

Le disque de pansement ou de mousse ainsi obtenu a été fixé par l'une de ses faces sur le plateau inférieur 1 du rhéomètre à l'aide d'un adhésif double face commercialisé par la société Plasto sous la référence P753. Puis le plateau supérieur 3 du rhéomètre a été mis en appui sur l'échantillon à la pression souhaitée (26 kPa) et simultanément on a appliqué à l'échantillon la déformation angulaire souhaitée (1%) à l'aide du rhéomètre. La mesure a été réalisée dans une chambre de mesure régulée à 35 °C pour simulerles conditions de température d'un patient alité. La sollicitation en régime dynamique a été réalisée par la mise en œuvre de fréquences de balayage du plateau mobile de 0,1 Hz, 1Hz et 10 Hz.

Le logiciel de pilotage du rhéomètre analyse, pour chaque couple de pression/cisaillement, le comportement viscoplastique de la mousse ou du pansement testé en calculant le module de cisaillement élastique **G'** et le module de perte ou dissipation en cisaillement **G "** pour chaque fréquence de sollicitation.

Les essais ont été réalisés trois fois pour chacune des trois fréquences retenues et on a retenu les valeurs moyennes de ces trois essais Le module de cisaillement complexe G* qui caractérise le comportement de l'échantillon testé, pour une sollicitation en cisaillement et pression en mode dynamique, est exprimé en Pascal (Pa) par l'équation [Math. 2] $G * = \sqrt{{G}^{′ 2} + {G}^{" 2}} .$

Mesure de la complaisance recouvrable de l'élément de décharge La mesure de la complaisance recouvrable de l'élément de décharge a été effectuée selon la méthode suivante.

Un échantillon de mousse de décharge seule ou complexée au film imperméable a été soumis à un couple pression/cisaillement à l'aide du même rhéomètre DHR2. On a utilisé ici un échantillon de 40 mm de diamètre de sorte que pour obtenir la contrainte axiale souhaitée de 28 kPa on a ajusté la force axiale du mobile à 3,5 N. On a simultanément appliqué une contrainte de cisaillement de 100 Pa pendant 6000 secondes puis la contrainte a été supprimée (ramenée à 0) pendant 1800 secondes. La fixation de l'échantillon est identique au protocole précédent et la température toujours régulée autour de 35°C.

La complaisance recouvrable exprimée en Pa⁻¹ a été calculée directement par le rhéomètre, à partir des valeurs mesurées de la déformation instantanée (DI) et de la déformation rémanente (DR) (lesdites valeurs étant exprimées sans unités) en divisant la différence entre ces valeurs (DI-DR) par la contrainte appliquée ici 100Pa.

Dans une première étape, on a ainsi testé les pansements suivants :
Pansement 1 : P1 Comfeel Plus Pressure Relief de la société Coloplast sans sa mousse de décharge
Pansement 2 : P2 Mepilex Border de la société Molnlycke
Pansement 3 : P3 Urgotul ABSORB BORDER de la société URGO Médical
Pansement 4 : P4 UrgoStart Plus Border de la société URGO Médical.

Le pansement Mepilex Border comprend deux couches absorbantes, à savoir une couche de mousse de polyuréthane hydrophile et un non-tissé de fibres super absorbantes.

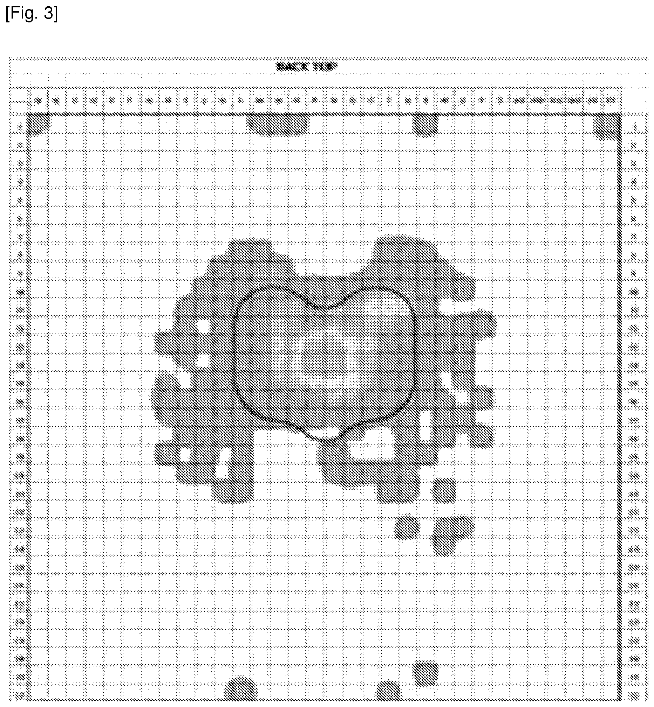

Le produit Urgotul Absorb Border (représenté sur la figure 3 de la demande de brevet WO2012 140377) comprend trois couches absorbantes, à savoir une mousse absorbante hydrophile en polyuréthane et deux non tissés absorbants contenant des fibres absorbantes et des particules de super absorbants. La mousse absorbante est une mousse de polyuréthane hydrophile de 2,6 mm d'épaisseur vendue par la société Advanced Medical Solutions sous la référence MCF 03. Chacun des non tissés absorbants est un non tissé airlaid (200 g/m 2) contenant des fibres absorbantes et des particules de polymère super-absorbant vendu par la société EAM Corporation sous la référence Novathin ^{®}.

Le produit Urgo start Plus Border (représenté sur la figure 1 de la demande de brevet WO2012 140378) comprend également trois couches absorbantes, à savoir un non tissé absorbant à base de fibres super absorbantes et de fibres thermoliantes et deux non tissés absorbants airlaid contenant des fibres et des particules de super absorbant. Le non tissé absorbant à base de fibres super absorbantes a un grammage de de 110 g/m2 et une épaisseur de 1,5 mm et contient 70% de fibres super absorbantes LANSEAL^{®} F commercialisées par la société TOYOBO CO LTD et 30 % de fibres thermoliantes bi-composant polyester/polyéthylène.
Chacun des deux autres non tissés absorbants est un non tissé airlaid (200 g/m²) contenant des fibres absorbantes et des particules de polymère super-absorbant vendu par la société EAM Corporation sous la référence Novathin.

Les résultats des mesures de G* sont rassemblés dans le tableau 1.

**[Table 1]**

| | G* 0,1 Hz en kPa | G* 1Hz en kPa | G* 10 Hz en kPa |
|---|---|---|---|
| Pansement 1 | 136 | 194 | 305 |
| Pansement 2 | 16 | 23 | 29 |
| Pansement 3 | 15 | 18 | 21 |
| Pansement 4 | 48 | 70 | 57 |

Ces résultats montrent que ces pansements présentent des comportements rhéologiques différents. Le pansement Comfeel Plus Pressure Relief affiche des valeurs de module de cisaillement complexe G*supérieures à 100 Pa pour toutes les fréquences ce qui n'est pas le cas des autres pansements étudiés.

Ces autres pansements peuvent être classés en deux catégories :
- les pansements 2 et 3 qui ont des valeurs du même ordre et qui comportent au moins deux couches absorbantes, dont une est une mousse absorbante ;
- le pansement 4 qui ne comprend aucune mousse absorbante et qui présente des valeurs de module de cisaillement complexe G*supérieures aux précédents.

Dans une deuxième étape, on a étudié des échantillons obtenus en ajoutant une mousse de décharge aux pansements préalablement testés comportant au moins deux couches absorbantes.

Les différentes mousses de décharge qui ont été utilisées pour ce test sont les suivantes :
Exemple 1 : Une mousse hydrophile de polyuréthane de la société Mayser dont la référence est Bluefoam TG - T100. Son épaisseur est de 6 mm et sa densité de 100 kg/m³.
Exemple 2 : Une mousse hydrophile de polyuréthane, complexée thermiquement avec un film, de la société INOAC dont la référence est INOAC 400/200. Son épaisseur est de 5,2 mm et sa densité de 147 kg/m³.
Exemple 3 : Une mousse hydrophile de polyuréthane, complexée thermiquement avec un film, de la société Advanced ATD Emolda dont la référence est ABDERMA D 170. Son épaisseur est de 5 mm et sa densité de 166 kg/m³.
Exemple 4 : Une mousse hydrophile de polyuréthane de la société Sentient Foams dont la référence est SENTIENT F11-5. Son épaisseur est de 5 mm et sa densité de 208 kg/m³.
Exemple 5 : La mousse hydrophobe utilisée dans le produit Comfeel Plus Pressure Relief. Son épaisseur est de 6 ,1 mm et sa densité de 33 kg/m³.
Exemple 6 : Une mousse hydrophile de polyuréthane de la société INOAC dont la référence est INOAC 1.0. Son épaisseur est de 5 mm et sa densité de 391 kg/m³.
Exemple 7 : La mousse de l'exemple 2 sans film.

Les caractéristiques de ces mousses sont rassemblées dans le tableau 2.

**[Table 2]**

| | Epaisseur en mm | Densité en kg/m³. | Complaisance Recouvrable | G* 0,1Hz en | G* 1Hz en | G* 10Hz en |
|---|---|---|---|---|---|---|
| | | | en Pa⁻¹ | kPa | kPa | kPa |
| Exemple1 | 6 | 100 | 1,4. 10⁻⁵ | 57 | 71 | 88 |
| Exemple2 | 5,2 | 147 | 3,1. 10⁻⁵ | 49 | 55 | 63 |
| Exemple3 | 5 | 166 | 1,4. 10⁻⁵ | 31 | 36 | 44 |
| Exemple4 | 5 | 208 | 1,0. 10⁻⁵ | 37 | 61 | 68 |
| Exemple5 | 6,1 | 33 | 0,86. 10⁻⁵ | 268 | 250 | 178 |
| Exemple6 | 5 | 391 | 0,65. 10⁻⁵ | 146 | 180 | 231 |
| Exemple7 | 5,2 | 147 | 3,2. 10⁻⁵ | 49 | 55 | 64 |

Les résultats ainsi obtenus ont permis de montrer que l'ajout d'un film de quelques dizaines de microns ne modifie pas les valeurs de G* et donc le comportement de la mousse. Ainsi, les résultats sont identiques aux 3 fréquences pour les exemples 2 et 7. La grande souplesse de ces films, qui sont des films de polyuréthane d'épaisseur comprise entre 10 et 40µm imperméables à l'eau et aux bactéries mais perméables à la vapeur d'eau, n'influe pas sur cette caractéristique de la mousse. Le film ne joue ni sur l'épaisseur ni sur la densité de la mousse vu son épaisseur et sa souplesse.

Ces résultats montrent donc que le module de cisaillement complexe G* des pansements modulables peut être calculé en testant la mousse seule aussi bien que la mousse complexé avec un film.

Ces résultats montrent encore qu'il est possible de classer ces mousses en 2 grandes catégories :
- celles des exemples 5 et 6 qui présentent des valeurs de G* supérieures à 100 ; et
- les autres mousses qui présentent des valeurs de G* inférieures à 100.

Cette classification est également vraie en se basant sur les valeurs de complaisance recouvrable de ces mousses. Ainsi, les mousses des exemples 5 et 6 sont caractérisées par une complaisance recouvrable inférieure à la valeur de 1. 10⁻⁵ Pa⁻¹ alors que toutes les autres mousses présentent une complaisance recouvrable supérieure à cette valeur.

Les pansements ayant fait l'objet de cette étude ont été préparés de la façon suivante.

L'élément de décharge utilisé est représenté sur la figure 2. Sa forme et sa surface sont similaires à celles d'un pansement sacrum habituellement utilisé et que cet élément de décharge recouvre entièrement. Il comprend deux zones :
- une partie périphérique 4 et
- une partie centrale 5 avec des parties détachables, qui dans cette étude sont formées de motifs hexagonaux (ou alvéoles) qui définissent une structure en nids d'abeille et qui ont été obtenus par découpe laser. La surface de chaque alvéole hexagonale était de 1.5 cm². Ces parties détachables forment des plots dont une quinzaine (ceux destinés à se trouver en vis-à-vis de la plaie) ont été retirés pour les tests. Bien évidemment la forme des parties détachables n'est pas limitée aux seuls motifs hexagonaux représentés et des formes différentes (rectangulaires, carrées ou circulaires) peuvent être envisagées.

La mousse de l'élément de décharge a été collée à la face visible d'un l'adhésif double face commercialisé par la société GERGONNE dont la référence est 01 E2970E55 22, puis le protecteur de la face cachée de l'adhésif double face a été retiré et l'ensemble collé sur le film de protection en polyuréthane du support siliconé du pansement UrgoStart Plus Border ou URGOTUL Absorb Border ou sur le film extérieur du pansement Mepilex Border.

On a ainsi testé les pansements modulables suivants :
Pansement M1 : Comfeel Plus Pressure Relief de la société Coloplast
Pansement M2 : pansement 2 + mousse exemple 2
Pansement M3 : pansement 3 + mousse exemple 2
Pansement M4 : pansement 3 + mousse exemple 6
Pansement M5 : pansement 3 + mousse exemple 5
Pansement M6 : pansement 4 + mousse exemple 6
Pansement M7 : pansement 4 + mousse exemple 5
Pansement M8 : pansement 4 + mousse exemple 2
Pansement M9 : pansement 4 + mousse exemple 3.

Les résultats des mesures de module de cisaillement complexe G* des pansements modulables testés sont rassemblés dans le tableau 3.

**[Table 3]**

| Pansement modulable | G* 0,1 Hz en kPa | G* 1Hz en kPa | G* 10Hz en kPa |
|---|---|---|---|
| M 1 | 128 | 167 | 218 |
| M 2 | 28 | 35 | 45 |
| M 3 | 26 | 32 | 38 |
| M 4 | 34 | 40 | 63 |
| M 5 | 41 | 48 | 57 |
| M 6 | 85 | 88 | 76 |
| M 7 | 89 | 91 | 76 |
| M 8 | 58 | 67 | 70 |
| M 9 | 39 | 39 | 56 |

Ces résultats montrent que ces pansements modulables, comme les pansements initiaux, présentent des comportements différents.

Le pansement Comfeel Plus Pressure Relief est à nouveau à part. Il affiche, à toutes les fréquences, des valeurs de G* supérieures à 100 Pa à la différence des autres pansements.

A partir de ces résultats, il est possible de classer ces autres pansements en quatre catégories qui recoupent les catégories des pansements sans mousse de décharge précédents.

Les pansements M2, M3, M4 et M5 dont la première couche absorbante est une mousse absorbante, qui peuvent être encore classés en deux sous-catégories :
- les pansements M2 et M3 qui présentent les modules complexe G* suivants :
   G* inférieur ou égal à 30 à 0,1 Hz ;
   G* inférieur ou égal à 40 à 1 Hz ;
   G* inférieur ou égal à 50 à 10 Hz ;
- les pansements M4 et M5 qui présentent les modules complexe G* suivants :
   G* supérieur à 30 à 0,1 Hz ;
   G* supérieur à 40 à 1 Hz ;
   G* supérieur à 50 à 10 Hz ;
- les pansements M6, M7, M8 et M9 dépourvus de mousse comme couche absorbante, qui peuvent être encore classés en deux sous-catégories :
- les pansements M8 et M9 qui présentent les modules complexe G* suivants :
   G* inférieur ou égal à 70 à 0,1 Hz ;
   G* inférieur ou égal à 70 à 1 Hz ;
   G* inférieur ou égal à 70 à 10 Hz ;
- les pansements M6 et M7 qui présentent les modules complexe G* suivants :
   G* supérieur à 70 à 0,1 Hz
   G* supérieur à 70 à 1 Hz
   G* supérieur à 70 à 10 Hz.

On constate que dans cette dernière catégorie, les pansements M6 et M7 présentent toujours des valeurs G* plus élevées que les autres. Ceci est cohérent puisque leur structure sans mousse est celle qui se rapproche le plus de celle du Pansement Comfeel Plus Pressure Relief. Le même phénomène est observé mais de façon plus atténuée pour les pansements M4 et M5 comprenant une mousse absorbante de la catégorie précédente.

Si l'on recoupe les valeurs de G* de ces pansements M 6 et M7 avec le tableau 2 qui résume les caractéristiques des mousses, on constate que les mousses des exemples 5 et 6 qui présentent des valeurs de complaisance recouvrable du même ordre conduisent à des valeurs de G* identiques. Elles ont donc un comportement similaire avec cette catégorie de pansements.

Pour corréler ces résultats avec le comportement du pansement en condition d'utilisation, le déposant a développé une technique permettant de mesurer et de visualiser les pressions qui s'appliquent sur le sacrum d'un sujet sans plaie portant chacun de ces pansements. La mise au point de cette technique a été complexe et elle implique des conditions de mise en œuvre précises pour obtenir des résultats exploitables.

Ces conditions de mise en œuvre sont les suivantes.

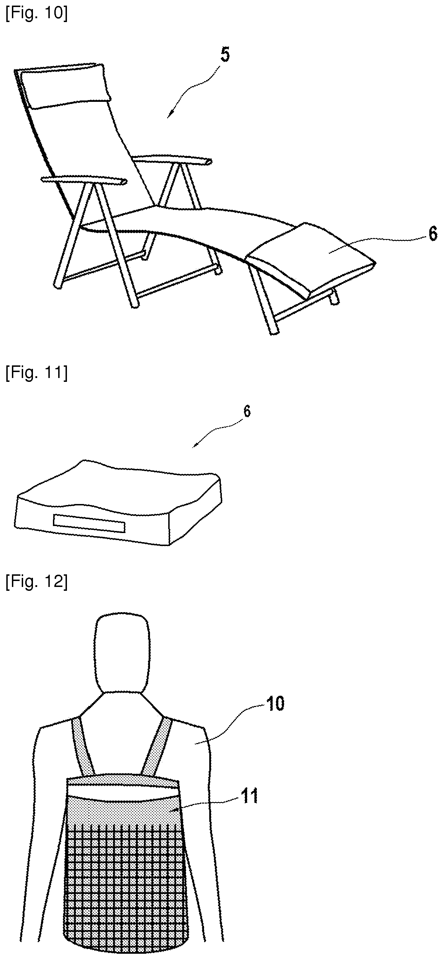

Dans les essais réalisés, on a utilisé un siège inclinable LAFUMA avec toile Rsxa Clip - Futura Batyline Iso Papageno, représenté schématiquement à la figure 10. Dans la mesure où la rigidité initiale de ce siège a tendance à diminuer au fur et à mesure que les testeurs se succèdent sur le siège et afin de limiter ces variations, le siège doit être conditionné avant chaque essai pour détendre le tissu. A cet effet, le testeur doit se positionner dans le siège en position inclinée (voir figure 10) et y rester pendant 10 min avant de se repositionner sur le siège pour le test.

Pour réaliser les mesures de pression on a utilisé une nappe textile de pression CONFORMat commercialisée sous la référence 5330 par la société MESCAN. Cette nappe est réglée et positionnée sur le testeur à l'aide d'un dispositif de fixation. Ce dernier comprend un système de « harnais » réglable et encadrant les épaules et le tour du torse/dos du testeur. Deux systèmes d'accroche ajoutés de part et d'autre de la nappe viennent se clipser sur le « harnais » pour positionner et maintenir la nappe en place sur le testeur de manière à ne pas avoir de plis et à ce qu'elle soit la plus centrée possible sur les zones à tester. On a représenté schématiquement à la figure 12 un testeur 10 portant cette nappe textile de pression 11.

Le testeur vient positionner ses pieds en appui sur la barre de pieds du siège 5 et s'assoit délicatement de façon à ce que la zone de son sacrum soit en contact avec la nappe de pression 11 tout en étant installé confortablement au plus loin dans le siège. Les pieds du testeur doivent bien rester en contact avec la barre du siège durant la phase d'assise. Le testeur est ensuite mis en inclinaison maximum dans le siège. L'objectif de cette position est de se placer dans des conditions dans lesquelles la pression appliquée sera la plus importante possible.

Une fois positionné, le testeur soulève son bassin de manière à ce que l'on puisse tendre la nappe sous lui et sur le siège. Une fois ce réglage terminé, on porte les jambes du testeur et les appose, au niveau des chevilles, sur un coussin d'aide à la prévention des escarres (coussin de décharge) commercialisé sous la dénomination Kalli de dimension 42 x 42 cm et d'épaisseur d'environ 8 cm. Ce coussin est représenté schématiquement à la figure 11. Les pieds du testeur sont alors positionnés dans les emplacements courbés du coussin, puis sont détendus sans mouvement de flexion du pied vers le testeur ni de pointe du pied. Le testeur place ensuite ses bras croisés sur son torse afin de garantir la répétabilité de la mesure.

Un trait au marqueur est ensuite ajouté sur les barres métalliques du siège à la hauteur du positionnement bas de la nappe. Cette marque permet de vérifier que le positionnement du testeur lors des différents essais est correct.

L'angle d'inclinaison entre le buste et les jambes, résultant de l'inclinaison du siège est estimé à 45-55°. Cet angle d'inclinaison est identique pour chaque testeur et choisi pour simuler le cas le plus défavorable pour la création d'une escarre. La position du testeur est enregistrée et conservée pour être répétée pour chacun des tests.

Les résultats des mesures sont obtenus à l'aide d'une interface électronique de Tekscan commercialisée par la société MESKAN et d'un ordinateur portable avec un logiciel I-scan et un fichier d'étalonnage. Ce fichier d'étalonnage sert à convertir exprimées sans unité les valeurs obtenues par les capteurs en pression. La nappe de capteurs est ainsi étalonnée préalablement avec un dynamomètre qui applique différentes forces, de sorte que le logiciel I-SCAN, connaissant la surface d'appui, peut convertir ces forces en pression.

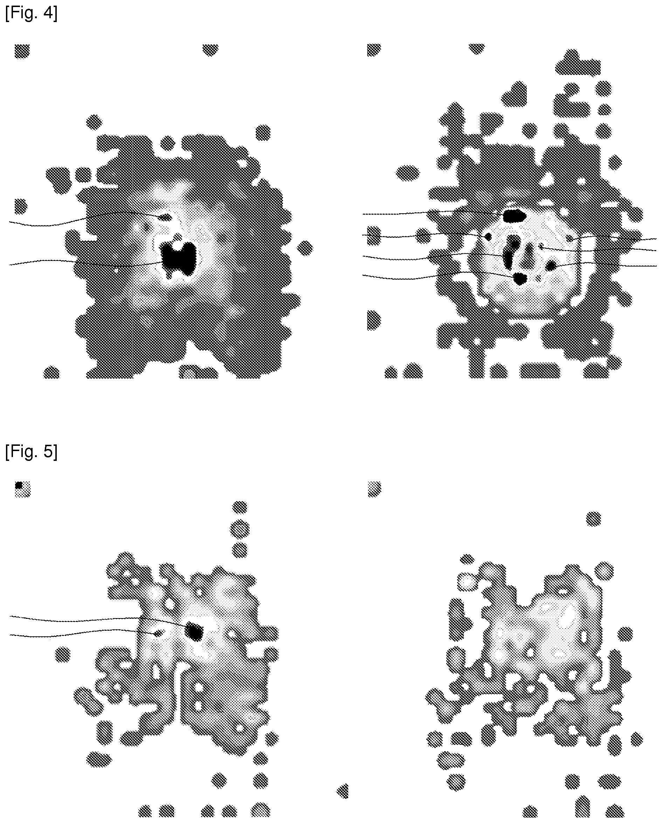

Les fichiers enregistrés dans le logiciel ont été traités et ont conduit à l'enregistrement des images qui sont présentées dans les figures 4 à 7 qui montrent la répartition des pressions sur le sacrum pour les différents pansements testés.

L'essai réalisé pour un testeur avec la nappe de capteurs sur le siège permet de localiser la zone où apparait une surpression sur le sacrum. L'image ainsi obtenue est qualifiée de témoin. Elle indique la zone de surpression où doit être positionné le pansement. A partir des mesures et des images réalisées on a ainsi créé un fichier EXCEL qui est représenté sur la figure 3.

Ce fichier montre la zone de pression à traiter avec un pansement fictif. Il comporte des échelles horizontale et verticale qui correspondent aux lignes et colonnes de la nappe. On dispose ainsi d'un moyen permettant de connaitre la position optimale dans laquelle le pansement à tester doit être positionné. Une fois cette détermination effectuée, la zone de décharge située au niveau du pansement fictif et définie par les échelles horizontales et verticales est utilisée pour positionner le pansement à tester et la zone à décharger sur la nappe de pression. La décharge est effectuée par retrait des parties détachables au cœur du pansement correspondant à la zone de décharge avant son positionnement sur le patient.

La figure 4 montre les résultats obtenus avec le pansement Comfeel Plus Pressure Relief (la partie de gauche montrant à titre comparatif, les résultats obtenus sans aucun pansement et la partie droite les résultats obtenus avec le pansement modulable après retrait des parties détachables).

Les images qui ont été obtenues par le déposant sont en couleur, chaque niveau de pression étant représenté par une couleur propre allant du rouge et orange pour les plus fortes pressions au vert et bleu pour les pressions les plus faibles en passant par le jaune pour des pressions moyennes.

Dans le cadre de la présente demande, les images étant fournies en noir et blanc, il a été choisi de ne distinguer que les seules zones de fortes pressions qui apparaissent en noir et qui sont désignées par des flèches.

Comme on peut le voir, sans aucun pansement, une pression importante s'applique sur la partie centrale de la surface du sacrum. Avec un pansement et après le retrait des parties détachables disposées au-dessus de cette zone de surpression, on constate une diminution de la pression au niveau de cette zone. En revanche, avec le pansement Comfeel Plus Pressure Relief, on peut observer une augmentation significative de la pression sur les bords de cette zone déchargée.

Une plaie cicatrisant à partir des bords, une telle augmentation de pression observée avec le pansement Comfeel Plus Pressure Relief montre que celui-ci n'est pas parfaitement adapté pour le traitement d'une escarre ou pour éviter son aggravation dans le temps.

Sans être lié par cette interprétation, il est probable que ce phénomène de report de pression sur les bords est lié à la nature de la mousse du pansement Comfeel Plus Pressure Relief qui n'est pas adaptée aux contraintes de cisaillement qui accompagnent la formation d'une escarre.

Un pansement efficace ne doit donc pas conduire à ce phénomène de report de pression sur les bords.

L'analyse du tableau 3 montre que les pansements M6 et M7 présentent des valeurs de G* similaires et les plus proches du pansement Comfeel Plus Pressure Relief. Il en est de même pour leurs mousses 5 et 6. Ces mousses ont donc été écartées dans le cadre de l'invention.

Il est à noter que la mousse 5 est hydrophobe et que les deux mousses 5 et 6 présentent des densités extrêmes par rapport aux autres mousses.

C'est ainsi que les inventeurs se sont orientés vers des mousses qui sont hydrophiles et qui présentent une densité comprise entre 70 et 250 kg/m³ et de préférence entre 90 et 220 kg/m³.

Pour vérifier que les mousses des exemples 1 à 4 soulagent la pression mais ne la reportent pas sur les bords, des tests complémentaires sur volontaires sains ont été réalisés en suivant le même protocole qu'avec le pansement Comfeel Plus Pressure Relief et en choisissant les mousses ayant les valeurs maximale et minimale de G* c'est-à-dire celle de l'exemple 1 et celle de l'exemple 3 ainsi que la mousse de l'exemple 2 qui présente une valeur intermédiaire.

Afin de diminuer le nombre de tests ces mousses ont été associées avec les 2 catégories de pansement P3 et P4. Dans tous ces tests, les parties détachables des mousses de décharge ont été retirées pour les mesures.

Les figures 5 à 7 illustrent les images de répartition des pressions obtenues pour ces pansements.

La figure 5 montre ainsi les résultats obtenus avec le pansement M8 qui comprend le pansement P4 associé à la mousse de décharge de l'exemple 2 (la partie de gauche montrant à titre comparatif, les résultats obtenus sans aucun pansement).

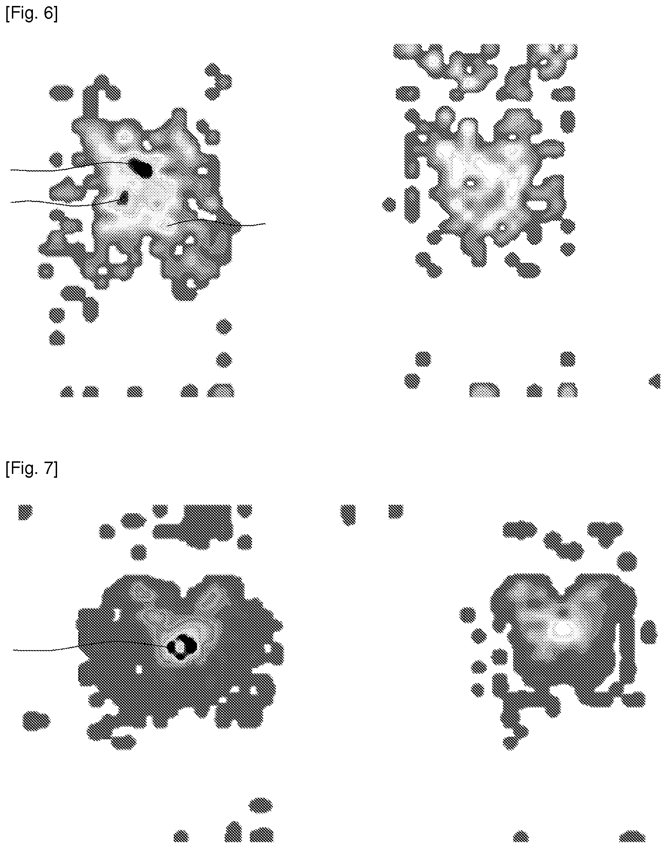

De même, la figure 6 montre les résultats obtenus avec le pansement M9 qui comprend le pansement P4 associé à la mousse de décharge de l'exemple 3 (la partie de gauche montrant à titre comparatif, les résultats obtenus sans aucun pansement).

De même encore, la figure 7 montre les résultats obtenus avec le pansement M10 qui comprend le pansement P3 associé à la mousse de décharge de l'exemple 1 (la partie de gauche montrant à titre comparatif, les résultats obtenus sans aucun pansement).

Ces images montrent que les pansements testés permettent de diminuer de façon significative la pression au niveau des zones de fortes pressions sans report de pression sur les bords de cette zone.

On observe en effet sur les images originales la disparition des zones rouges et oranges qui définissent les plus fortes pressions et seules subsistent quelques zones jaunes ponctuelles (le reste de l'image étant bleu).

Comme en ce qui concerne la figure 4, il a été choisi, sur ces figures en noir et blanc, de ne distinguer que les seules zones de fortes pressions qui apparaissent en noir et qui sont désignées par des flèches.

Ces résultats ont permis de confirmer que les mousses hydrophiles présentant un module de cisaillement complexe G* compris :
- entre 20 et 70 kPa et de préférence entre 30 et 60 kPa à une fréquence de 0,1 Hz
- entre 30 et 90 kPa et de préférence entre 35 et 80 kPa à une fréquence à une fréquence de 1 Hz
- entre 40 et 100 kPa et de préférence entre 40 et 90 kPa à une fréquence de 10 Hz et de préférence une valeur de complaisance recouvrable comprise entre 1 et 4 .10⁻⁵ Pa⁻¹ permettent de réaliser des pansements adaptés au traitement des escarres et/ou prévenant leur aggravation.

Dans la mesure où les essais qui viennent d'être rapportés ont été réalisés sur un sujet sans plaie, des essais additionnels de modélisation ont été conduits pour confirmer les résultats obtenus dans le cas d'une plaie de stade 1 ou 2.

Dans ce contexte, l'action du cisaillement sur les tissus mous dans le cas d'une plaie de stade 2 a été évaluée.

Il est connu d'utiliser la technique de modélisation par éléments finis pour étudier le biomécanisme de création des escarres et évaluer la performance des pansements. Cependant, cette technique permet de faire des comparaisons entre différents pansements mais ne permet pas de quantifier la déformation de cisaillement appliquée sur les tissus. De plus cette technique de modélisation n'inclut pas la présence d'une plaie.

Pour tenter de quantifier les déformations en cisaillement qui s'appliquent sur les tissus mous au niveau du sacrum, une première expérimentation a été réalisée sur un volontaire sans plaie avec le pansement modulable M8 selon l'invention. Cette quantification a permis de « chiffrer » et donc d'évaluer l'efficacité du pansement modulable. On a ensuite simulé un modèle de plaie de stade 2 et chiffré l'impact de l'élément de décharge sur les déformations internes dans les tissus mous d'une plaie de stade 2 et de leur zone périlésionnelle au niveau du sacrum.

Les différentes étapes suivantes ont ainsi été mises en œuvre:
a) modélisation des tissus mous du sacrum et d'une plaie de stade 2.

Cette étude a été réalisée sur la base des données recueillies sur un volontaire sans plaie âgé de 40 ans dont le poids et la taille étaient respectivement de 94 Kg et 173 cm.

Le modèle paramétrique de la zone sacrée est composé de deux épaisseurs de tissus mous : le derme et le tissu adipeux.

Les données recueillies sur le volontaire ont permis d'établir que l'épaisseur du derme est de 1,3 mm et l'épaisseur du tissu adipeux de 13,3 mm sous la crête osseuse et de 22,3 mm à l'extérieur de la crête osseuse.

La géométrie du sacrum retenue a été importée sur Ansys Mechanical APDL (Ansys, Canonburg Pennsylvania USA) pour être maillée avec des éléments linéaires hexaédriques (SOLID185) en formulation hybride pression-déplacement. Le nombre total d'éléments des tissus mous est de 15 936. Les dimensions de la plaie ont été définies par rapport aux données d'un sondage réalisé par le déposant auprès du personnel soignant en supposant la plaie cylindrique avec des bordures lissées. Le rayon du fond de la plaie est ainsi de 13,0 mm tandis que la profondeur est de 1,3 mm pour une plaie de stade 2. Une rigidification des tissus mous autour de la plaie a été prise en compte. Trois zones distinctes ont ainsi été définies dans les modèles pour la peau comme pour le tissu adipeux : zone de tissus rigides, zone de tissus médians et zone de tissus mous. L'épaisseur de ces trois zones a été fixée arbitrairement à 1,3mm. Les modèles ont été maillés sous Ansys Mechanical APDL avec des éléments hexaédriques (SOLID185) en formulation hybride pression-déplacement. Le nombre total des éléments hexaédriques de cette simulation de la plaie était de 18664.

b) modélisation du pansement modulable avec son matériau de décharge Le pansement modulable avec son élément de décharge a été modélisé par un cylindre bi-couche de rayon 125,0 mm. La hauteur mesurée du pansement P4 (Urgostart Plus Border) était de 3,5 mm et cette hauteur a été retenue pour la modélisation du pansement. Pour l'élément de décharge (mousse de l'exemple 2) une épaisseur de 5,2 mm a été mesurée et retenue. Dans cette étude, la couche de décharge a été volontairement vidée d'un certain nombre de ses parties détachables pour modéliser la décharge. Le nombre d'alvéoles ainsi retirées correspondait approximativement aux dimensions des parties retirées lors des tests *in vivo* préalablement réalisés soit 20,0 x 20,0 mm². Le pansement P4 et l'élément de décharge ont été maillées avec des éléments hexaédriques de type (SOLID185) sur Ansys Mechanical APDL. Le nombre total des éléments hexaédriques du pansement P4 était de 2476 et celui de l'élément de décharge de 3612.

c) modélisation de l'interaction du pansement modulable M8 avec son matériau de décharge avec une plaie de stade 2

On a étudié avec cette modélisation, les configurations sans pansement, avec pansement sans élément de décharge, avec pansement modulable pourvu de son élément de décharge.

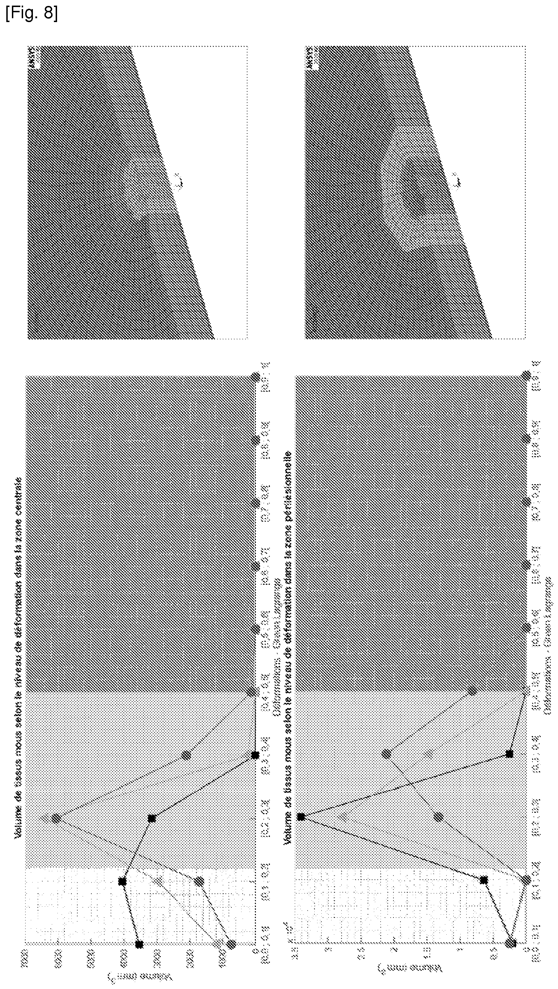

La figure 8 illustre la modélisation obtenue avec Ansys Mechanical APDL. Elle représente les déformations maximales de cisaillement (selon la définition de Green-Lagrange) dans les tissus mous de la zone centrale et de la zone perilésionnelle du modèle avec une plaie de stade 2.

La courbe avec des cercles représente la situation sans pansement.

La courbe avec les triangles représente la situation avec le pansement P4 seul (sans élément de décharge). La courbe avec les carrés représente la situation avec le pansement modulable M8.

La partie haute de la figure représente l'évaluation des contraintes de cisaillement au centre de la plaie et la partie basse de cette figure représente l'évaluation des contraintes de cisaillement sur le pourtour de la plaie (zone périlésionnelle). Les parties se situant à droite sur la figure illustrent la zone étudiée. On voit en abscisse la déformation maximale en cisaillement et en ordonnée le volume de tissus mous qui subit ces déformations. Les plages de déformations de couleur grise de plus en plus marquée de la gauche vers la droite (vertes, oranges et rouges sur les graphes originaux) indiquent respectivement les zones à faible, moyen et fort risque lésionnel. Plus on se déplace vers la droite plus ce risque est important. Il est à noter que dans le cas du pourtour de la plaie l'échelle des volumes est différente de l'échelle des volumes dans le cas de la partie centrale de la plaie, ce qui montre que les phénomènes y sont moins importants.

On constate également que l'ajout du pansement modulable avec son élément de décharge conduit à une diminution de ces déformations dans les deux zones d'intérêt que sont i) la zone centrale, considérée comme la zone la plus sensible de former une escarre puisque située directement sous la proéminence osseuse et ii) la zone périlésionnelle qui est constituée des tissus mous à moins de 15,0 mm de la zone centrale.

Par ailleurs, on constate que les valeurs de déformations maximales sont majoritairement inférieures à 0,5 quand on ajoute l'élément de décharge. Cette valeur est un bio marqueur numérique important dans la prévention des escarres et on considère qu'une valeur au-delà de 0,65 doit être évitée.

On constate enfin qu'avec le pansement modulable pourvu de son élément de décharge les courbes sont déplacées vers la gauche qui est la zone où les risques lésionnels sont les plus faibles.

Ainsi avec l'élément de décharge on passe d'un pic de déformation de 0,2 à 0,1 sur la zone centrale, et sur la zone périlésionnelle on n'augmente pas le pic de déformation qui reste inchangé à 0,3, c'est-à-dire une valeur bien inférieure à la valeur limite de 0,65 et dans une zone qui n'est pas à risque. Ces résultats confirment que le pansement et la mousse sélectionnée sont efficaces au centre de la plaie et ne conduisent à aucun report de contrainte sur le pourtour de la plaie.

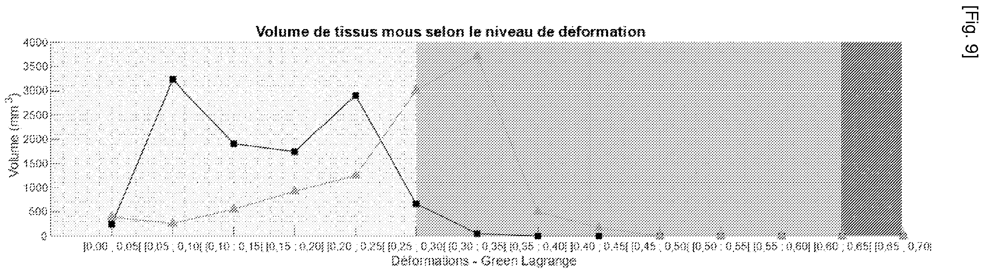

La figure 9 illustre le même type de représentation mais sur l'ensemble des deux zones étudiées avec et sans le pansement modulable M8.

La courbe avec les triangles représente la situation sans aucun pansement.

La courbe avec les carrés représente la situation avec le pansement M8.

On visualise encore mieux l'action positive du pansement selon l'invention.

On constate en effet sur cette figure la diminution de la hauteur des pics et surtout le déplacement des courbes vers la gauche dans la zone où le risque de lésion est le plus faible.

L'ensemble des études réalisées par le déposant montre que les pansements de l'invention sont utiles dans le traitement des escarres, en particulier des escarres du sacrum et de leur aggravation.

## Revendications

1. Pansement modulable utilisable dans le traitement chez un patient d'une escarre, en particulier une escarre du sacrum, ledit pansement comprenant :
- au moins deux couches absorbantes dont une destinée à venir au contact de la plaie; et
- une couche de mousse de décharge sous forme d'une mousse hydrophile de polyuréthane présentant une densité comprise entre 70 et 250 kg/m³, une épaisseur comprise entre 5 et 10 mm et comportant un ensemble (5) de parties contigües indépendamment détachables, lesdites parties destinées à se trouver en vis-à-vis de la plaie ou d'une partie de celle-ci, en position d'utilisation, et pouvant étant retirés lors de l'utilisation chez le patient afin de réduire ou supprimer la pression susceptible de s'exercer sur cette zone,
**caractérisé en ce que** ladite mousse de décharge est une mousse hydrophile qui présente une épaisseur d'au moins 5 mm et un module de cisaillement complexe G*, mesuré selon la méthode indiquée dans description, compris :
- entre 20 et 70 kPa, et de préférence entre 30 et 60 kPa, à une fréquence de 0,1 Hz
- entre 30 et 90 kPa, et de préférence entre 35 et 80 kPa, à une fréquence à une fréquence de 1 Hz
- entre 40 et 100 kPa, et de préférence entre 40 et 90 kPa, à une fréquence de 10 Hz,
et **en ce qu'**un film est fixé sur la face de ladite couche de mousse de décharge destinée à être la plus éloignée de la pluie en position d'utilisation, avant la formation desdites parties contigües indépendamment détachables, ledit film étant un film de protection perméable à la vapeur d'eau et imperméable aux liquides.

2. Pansement modulable selon la revendication 1 **caractérisé en ce que** la mousse de décharge hydrophile présente une densité comprise entre 90 et 220 kg/m³.

3. Pansement modulable selon l'une des revendications précédentes **caractérisé en ce que** la mousse de décharge présente une épaisseur entre 5 et 7 mm.

4. Pansement modulable selon l'une des revendications précédentes **caractérisé en ce que** la mousse présente une complaisance recouvrable, mesurée selon la méthode indiquée dans la description, supérieure ou égale à 1.10⁻⁵ Pa⁻¹ de préférence comprise entre 1. 10⁻⁵ Pa⁻¹ et 4. 10⁻⁵ Pa⁻¹.

5. Pansement modulable selon l'une des revendications précédentes **caractérisé en ce qu'**il présente un coefficient de friction mesurée selon la méthode indiquée dans la description, mesuré côté film, inférieur à 1 et de préférence compris entre 0,4 et 0,8.

6. Pansement modulable selon l'une des revendications précédentes **caractérisé en ce qu'**il comprend au moins deux couches absorbantes choisies parmi les mousses hydrophiles absorbantes, les non tissés absorbants contenant des particules super absorbantes, une couche comprenant des particules de polymères super absorbants entre deux non tissés et les non tissés à base de fibres super absorbantes.

7. Pansement modulable selon la revendication 6 **caractérisé en ce qu'**il comprend une couche de mousse absorbante hydrophile et une ou plusieurs couches de non tissé de fibres absorbantes incorporant des particules de polymère super absorbant ou une couche comprenant des particules de polymères super absorbants entre deux non tissés ou un non tissé à base de fibres super absorbantes.

8. Pansement modulable selon la revendication 6 **caractérisé en ce qu'**il comprend un non tissé à base de fibres super absorbantes et une ou plusieurs couches de non tissé de fibres absorbantes incorporant des particules de super absorbant ou une couche comprenant des particules de polymères super absorbants entre deux non tissés.

9. Pansement modulable selon la revendication 7 ou 8 **caractérisé en ce que** la couche de non tissé précité présente une épaisseur comprise entre 0,5 et 3 mm, et/ou un grammage compris entre 200 et 800 g/m².

10. Mousse de décharge utilisable dans le traitement d'une escarre, en particulier d'une escarre du sacrum, ladite mousse comportant un ensemble (5) de parties contigües indépendamment détachables, lesdites parties destinées à se trouver en vis-à-vis de la plaie ou d'une partie de celle-ci, en position d'utilisation, étant aptes à être retirés afin de réduire ou supprimer la pression susceptible de s'exercer sur cette zone, **caractérisé en ce que** ladite mousse de décharge est une mousse polyuréthane hydrophile qui présente une épaisseur d'au moins 5 mm et d'au plus 10 mm, une densité comprise entre 70 et 250 kg/m³ et un module de cisaillement complexe G**, mesuré selon la méthode indiquée dans la description, compris :
- entre 20 et 70 kPa, et de préférence entre 30 et 60 kPa, à une fréquence de 0,1 Hz
- entre 30 et 90 kPa, et de préférence entre 35 et 80 kPa, à une fréquence à une fréquence de 1 Hz
- entre 40 et 100 kPa, et de préférence entre 40 et 90 kPa, à une fréquence de 10 Hz et **en ce que** ladite couche de mousse de décharge est recouverte sur une face d'un film de protection perméable à la vapeur d'eau et imperméable aux liquides.

## Patentansprüche

1. Einstellbarer Verband zur Verwendung bei der Behandlung eines Patienten mit einem Dekubitalgeschwür, insbesondere einem Dekubitus (Druckgeschwür) am Kreuzbein, wobei der Verband Folgendes umfasst:
- mindestens zwei absorbierende Schichten, von denen eine dazu bestimmt ist, mit der Wunde in Kontakt zu kommen; und
- eine Schicht aus Entlastungsschaum in Form eines hydrophilen Polyurethanschaums mit einer Dichte zwischen 70 und 250 kg/m³, einer Dicke zwischen 5 und 10 mm und umfassend einen Satz (5) von unabhängig ablösbaren aneinander angrenzenden Teilen, wobei die Teile dazu bestimmt sind, in der Gebrauchsstellung der Wunde oder einem Teil davon zugewandt zu sein, und während des Gebrauchs am Patienten entfernt werden zu können, um jeglichen potenziell entstehenden Druck auf diese Zone zu verringern oder zu beseitigen;
**dadurch gekennzeichnet, dass** der Entlastungsschaum ein hydrophiler Schaum mit einer Dicke von mindestens 5 mm und einem komplexen Schermodul G* ist, gemessen nach dem in der Beschreibung angegebenen Verfahren, betragend:
- zwischen 20 und 70 kPa, vorzugsweise zwischen 30 und 60 kPa, bei einer Frequenz von 0,1 Hz
- zwischen 30 und 90 kPa, vorzugsweise zwischen 35 und 80 kPa, bei einer Frequenz von 1 Hz
- zwischen 40 und 100 kPa, vorzugsweise zwischen 40 und 90 kPa, bei einer Frequenz von 10 Hz,
und dadurch, dass vor der Bildung der unabhängig ablösbaren aneinander angrenzenden Teilen eine Folie auf der Fläche der genannten Schicht aus Entlastungsschaum angebracht wird, die in der Gebrauchsstellung am weitesten vom der Wunde entfernt zu liegen kommt, wobei die Folie eine wasserdampfdurchlässige und flüssigkeitsundurchlässige Schutzfolie ist.

2. Einstellbarer Verband nach Anspruch 1, **dadurch gekennzeichnet, dass** der hydrophile Entlastungsschaum eine Dichte zwischen 90 und 220 kg/m³ aufweist.

3. Einstellbarer Verband nach einem der vorhergehenden Ansprüche, **dadurch gekennzeichnet, dass** der Entlastungsschaum eine Dicke zwischen 5 und 7 mm aufweist.

4. Einstellbarer Verband nach einem der vorhergehenden Ansprüche, **dadurch gekennzeichnet, dass** der Schaum eine nach dem in der Beschreibung angegebenen Verfahren gemessene erzielbare Nachgiebigkeit größer oder gleich 1.10⁻⁵ Pa⁻¹, vorzugsweise zwischen 1. 10⁻⁵ Pa⁻¹ und 4. 10⁻⁵ Pa⁻¹ aufweist.

5. Einstellbarer Verband nach einem der vorhergehenden Ansprüche, **dadurch gekennzeichnet, dass** er einen nach dem in der Beschreibung angegebenen Verfahren gemessenen, folienseitig gemessenen Reibungskoeffizienten von weniger als 1, und vorzugsweise zwischen 0,4 und 0,8 aufweist.

6. Einstellbarer Verband nach einem der vorhergehenden Ansprüche, **dadurch gekennzeichnet, dass** er mindestens zwei absorbierende Schichten, die ausgewählt sind aus absorbierenden hydrophilen Schäumen, absorbierenden Vliesen mit superabsorbierenden Partikeln, einer Schicht, umfassend superabsorbierende Polymerpartikel zwischen zwei Vliesen, und Vliesen auf Basis superabsorbierender Fasern, umfasst.

7. Einstellbarer Verband nach Anspruch 6, **dadurch gekennzeichnet, dass** er eine Schicht aus hydrophilem absorbierendem Schaum und eine oder mehrere Schichten aus Vliesen absorbierender Fasern, enthaltend Partikel aus superabsorbierendem Polymer, oder eine Schicht, umfassend Partikel aus superabsorbierenden Polymeren zwischen zwei Vliesen, oder ein Vlies auf Basis superabsorbierender Fasern umfasst.

8. Einstellbarer Verband nach Anspruch 6, **dadurch gekennzeichnet, dass** er ein Vlies auf Basis superabsorbierender Fasern und eine oder mehrere Schichten Vlies aus absorbierenden Fasern, enthaltend Partikel aus Superabsorbens, oder eine Schicht, umfassend Partikel aus superabsorbierenden Polymeren zwischen zwei Vliesen, umfasst.

9. Einstellbarer Verband nach Anspruch 7 oder 8, **dadurch gekennzeichnet, dass** die oben genannte Vliesschicht eine Dicke zwischen 0,5 und 3 mm und/oder ein Flächengewicht zwischen 200 und 800 g/m² aufweist.

10. Entlastungsschaum zur Verwendung bei der Behandlung eines Dekubitalgeschwürs, insbesondere eines Dekubitus am Kreuzbein, wobei der Schaum einen Satz (5) von unabhängig ablösbaren aneinander angrenzenden Teilen umfasst, wobei die Teile dazu bestimmt sind, in der Gebrauchsstellung der Wunde oder einem Teil davon zugewandt zu sein, und entfernt werden können, um jeglichen potenziell entstehenden Druck auf diese Zone zu verringern oder zu beseitigen, **dadurch gekennzeichnet, dass** der Entlastungsschaum ein hydrophiler Polyurethanschaum ist, aufweisend eine Dicke von mindestens 5 mm und höchstens als 10 mm, eine Dichte zwischen 70 und 250 kg/m³ und ein komplexes Schermodul G**, gemessen nach dem in der Beschreibung angegebenen Verfahren, betragend:
- zwischen 20 und 70 kPa, vorzugsweise zwischen 30 und 60 kPa, bei einer Frequenz von 0,1 Hz
- zwischen 30 und 90 kPa, vorzugsweise zwischen 35 und 80 kPa, bei einer Frequenz von 1 Hz
- zwischen 40 und 100 kPa, vorzugsweise zwischen 40 und 90 kPa, bei einer Frequenz von 10 Hz, und dadurch, dass die genannte Schicht aus Entlastungsschaum einseitig mit einer wasserdampfdurchlässigen und flüssigkeitsundurchlässigen Schutzfolie bedeckt ist.

## Claims

1. Modular dressing that can be used in the treatment of a patient for a pressure ulcer, in particular a sacral pressure ulcer, said dressing comprising:
- at least two absorbent layers, one of which is intended to come into contact with the wound; and
- a layer of offloading foam in the form of a hydrophilic polyurethane foam having a density of between 70 and 250 kg/m³, a thickness of between 5 and 10 mm and comprising a set (5) of independently detachable contiguous parts, said parts intended to face the wound or a part thereof, in the position of use, and being able to be removed during use on the patient in order to reduce or eliminate the pressure which is liable to be exerted on this area,
**characterized in that** said offloading foam is a hydrophilic foam which has a thickness of at least 5 mm and a complex shear modulus G*, measured according to the method indicated in the description, of:
- between 20 and 70 kPa, and preferably between 30 and 60 kPa, at a frequency of 0.1 Hz
- between 30 and 90 kPa, and preferably between 35 and 80 kPa, at a frequency of 1 Hz
- between 40 and 100 kPa, and preferably between 40 and 90 kPa, at a frequency of 10 Hz,
and **in that** a film is fastened to the face of said layer of offloading foam that is intended to be furthest away from the wound in the position of use, prior to the formation of said independently detachable contiguous parts, said film being a protective film permeable to water vapour and impermeable to liquids.

2. Modular dressing according to Claim 1, **characterized in that** the hydrophilic offloading foam has a density of between 90 and 220 kg/m³.

3. Modular dressing according to either of the preceding claims, **characterized in that** the offloading foam has a thickness of between 5 and 7 mm.

4. Modular dressing according to any of the preceding claims, **characterized in that** the foam has a recoverable compliance, measured according to the method indicated in the description, of greater than or equal to 1.10⁻⁵ Pa⁻¹, preferably of between 1. 10⁻⁵ Pa⁻¹ and 4. 10⁻⁵ Pa⁻¹.

5. Modular dressing according to any of the preceding claims, **characterized in that** it has a coefficient of friction, measured according to the method indicated in the description, measured on the film side, of less than 1 and preferably of between 0.4 and 0.8.

6. Modular dressing according to any of the preceding claims, **characterized in that** it comprises at least two absorbent layers chosen from absorbent hydrophilic foams, absorbent nonwovens containing superabsorbent particles, a layer comprising particles of superabsorbent polymers between two nonwovens and nonwovens based on superabsorbent fibres.

7. Modular dressing according to Claim 6, **characterized in that** it comprises a layer of hydrophilic absorbent foam and one or more layers of absorbent fibre nonwoven incorporating particles of superabsorbent polymer or a layer comprising particles of superabsorbent polymers between two nonwovens or a nonwoven based on superabsorbent fibres.

8. Modular dressing according to Claim 6, **characterized in that** it comprises a nonwoven based on superabsorbent fibres and one or more layers of absorbent fibre nonwoven incorporating superabsorbent particles or a layer comprising particles of superabsorbent polymers between two nonwovens.

9. Modular dressing according to Claim 7 or 8, **characterized in that** the aforementioned nonwoven layer has a thickness of between 0.5 and 3 mm, and/or a basis weight of between 200 and 800 g/m².

10. Offloading foam that can be used in the treatment of a pressure ulcer, in particular a sacral pressure ulcer, said foam comprising a set (5) of independently detachable contiguous parts, said parts intended to face the wound or a part thereof, in the position of use, being capable of being removed in order to reduce or eliminate the pressure which is liable to be exerted on this area, **characterized in that** said offloading foam is a hydrophilic polyurethane foam which has a thickness of at least 5 mm and of at most 10 mm, a density of between 70 and 250 kg/m³ and a complex shear modulus G**, measured according to the method indicated in the description, of:
- between 20 and 70 kPa, and preferably between 30 and 60 kPa, at a frequency of 0.1 Hz
- between 30 and 90 kPa, and preferably between 35 and 80 kPa, at a frequency of 1 Hz
- between 40 and 100 kPa, and preferably between 40 and 90 kPa, at a frequency of 10 Hz, and **in that** said layer of offloading foam is covered on one face with a protective film permeable to water vapour and impermeable to liquids.
